# EUROPÄISCHE PATENTSCHRIFT

(11) **EP 2 836 818 B1**
(45) Veröffentlichungstag und Bekanntmachung des Hinweises auf die Patenterteilung: **08.06.2016**
(21) Anmeldenummer: 13715224.5
(22) Anmeldetag: 10.04.2013
(51) Int. Cl.: G01N 21/35

(54) **GASDETEKTORSYSTEM**
GAS DETECTOR SYSTEM
SYSTÈME DE DÉTECTION DE GAZ

(30) Priorität: 14.04.2012 DE 102012007561
(43) Veröffentlichungstag der Anmeldung: 18.02.2015
(73) Patentinhaber: Dräger Safety AG & Co. KGaA, 23560 Lübeck (DE)
(72) Erfinder: TRÖLLSCH, Arne, 23568 Lübeck (DE)
(74) Vertreter: Guthöhrlein, Gerhard
(86) Internationale Anmeldenummer: PCT/EP2013/057474
(87) Internationale Veröffentlichungsnummer: WO 2013/153106

(56) Entgegenhaltungen:
- US-A- 5 591 975
- US-A- 5 677 762
- US-A1- 2003 191 393
- US-A1- 2011 268 453

## Beschreibung

Die vorliegende Erfindung betrifft ein Gasdetektorsystem mit einem Sender mit einer Lichtquelle, von der ein Analyselichtstrahl emittiert wird, und mit einer Sender-Linsenordnung, die ausgestaltet ist, den Analyselichtstrahl entlang einer Emissionsrichtung zu fokussieren, sowie mit einem Empfänger mit einem Analysedetektor und einem Referenzdetektor.

Die Analyse von Gasgemischen hat sowohl in der Prozessleit- und Überwachungstechnik als auch in der Umweltanalytik eine zunehmende Bedeutung erlangt. Zudem steigen die Ansprüche an solche Messsysteme zur Gasanalyse hinsichtlich Messempfindlichkeit, Langzeitstabilität, Selektivität, wie auch die Anforderungen in Bezug auf die Wartungsintervalle und die Lebensdauer der Messsysteme mit dem zunehmenden Automatisierungsgrad in der Industrie und Umweltüberwachung. Um beispielsweise in der Umweltanalytik oder in der Überwachungstechnik größerer Industrieanlagen im Fehlerfall austretende Gase möglichst schnell erkennen zu können, ist es wünschenswert, die zu überwachenden Bereiche möglichst engmaschig und großflächig abzudecken. Dazu kann eine große Anzahl lokal eng begrenzt sensitiver Sensoren dienen, die über Datenverbindungen miteinander verbunden sein können.

Weitaus vorteilhafter und effektiver sind jedoch optisch abbildende Gassensoren, bei denen das abgestrahlte Licht über große Messstrecken ausgerichtet wird und wobei die Absorption des Lichts den gasartspezifischen Messeffekt darstellt. Solche Systeme erlauben Angaben über die mittlere Gaskonzentration in der Messstrecke und eine Überwachung größerer Bereiche.

Derartige Gasdetektorsysteme mit einem Sender, einem Empfänger und einer dazwischen liegenden freien Messstrecke, entlang derer die Gaszusammensetzung bzw. die Konzentration einer bestimmten Gaskomponente erfasst wird, werden gewöhnlich als Open-Path-Systeme bezeichnet, wobei der Abstand zwischen Sender und Empfänger, also die Länge der freien Messstrecke, im Bereich von 200 m liegen kann. Dies macht es erforderlich, dass der Sender und der Empfänger exakt zueinander ausgerichtet sind, damit der von dem Sender ausgesandte Analyselichtstrahl auch tatsächlich in vollem Umfang auf die Optik des Empfängers trifft. Aber selbst wenn dies der Fall ist, ist es darüber hinaus erforderlich, dass der Analyselichtstrahl exakt an der richtigen Stelle auf den Empfänger trifft, damit sichergestellt ist, dass eine möglichst große Intensität in den im Empfänger vorhandenen Detektoren nachgewiesen werden kann. Wenn also Sender und/oder Empfänger nicht richtig ausgerichtet sind, kann das System nicht zuverlässig bzw mit der gewünschten Empfindlichkeit arbeiten.

Ein solches Gassensorsystem mit einer offenen Messstrecke mit einem Laser als Lichtquelle und einem Empfangselement in einem gemeinsamen Gehäuse ist beispielsweise aus der U.S. 5,339,155 bekannt, worin beschrieben ist, dass Licht mit Hilfe eines halbdurchlässigen Spiegels und einen schräg stehenden Spiegel auf einen Hohlspiegel und von dort als paralleles Strahlenbündel durch die offene Messstrecke hindurch auf einen entfernt stehenden Reflektor gerichtet und abgestrahlt wird und von dort zum Empfangselement zurück in das Gehäuse reflektiert wird.

Aus der U.S. 6,538,728 ist eine Anordnung mit einem optischen Gasmesssystem mit einer offenen Messstrecke, einem Sender und einem Empfänger bekannt, bei der im Sender eine Messlichtquelle einen Analysestrahl in eine offene Messstrecke emittiert, der Analysestrahl die offenen Messstrecke durchquert und ein Detektor im Empfänger den Analysestrahl registriert. Auf Basis des vom Detektor registrierten Analysestrahls wird eine Gaskonzentration eines Zielgases ermittelt. Weiterhin sind in der U.S. 6,538,728 zwei optische oder telemetrische Kommunikationskanäle offenbart, auf denen ein bidirektionaler Datenaustausch zwischen Sender und Empfänger ermöglicht ist. Mit Hiife dieses Datenaustausches wird ermöglicht, aus den Empfangsdaten, wie empfangene Lichtintensität, Informationen bezüglich der Ausrichtung von Sender und Empfänger am Sender und am Empfänger mittels Datenkommunikation verfügbar zu machen und damit eine Dejustierung zu erkennen und eine Korrektur der Justage zu unterstützen.

Aus der U.S. 5,591,975 ist ein Gassensorsystem bekannt, bei dem der Lichtstrahl nach Durchlaufen eines zu beobachtenden Bereiches im Empfänger homogenisiert wird, wobei zur Homogenisierung ein Linsensystem umfassend u.a. eine plankonvexe Linse mit einem Array von flachen Facetten benutzt wird.

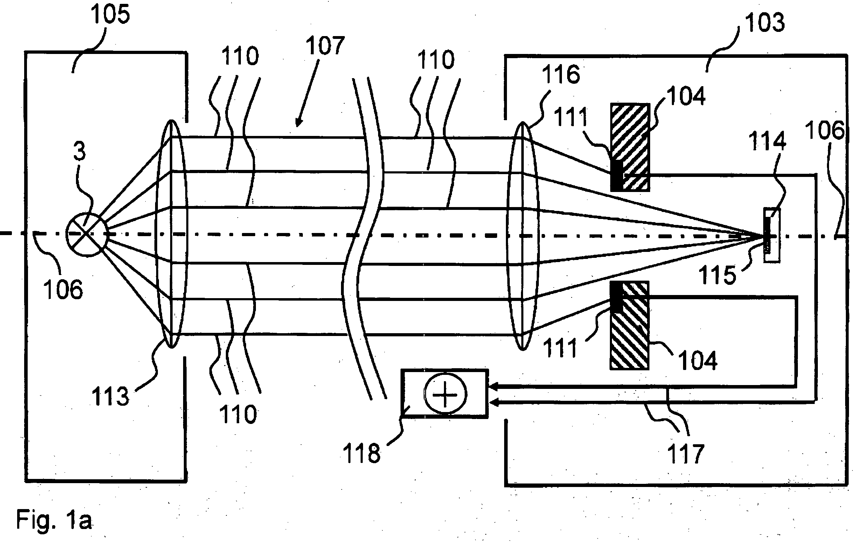

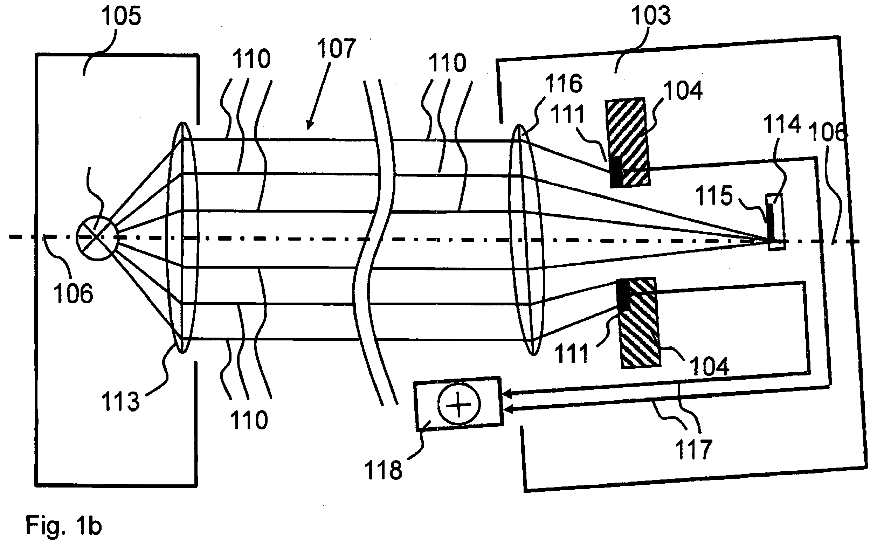

Des Weiteren sind aus dem Stand der Technik (Figuren 1a und 1b) Anordnungen bekannt, bei denen eine Justagevorrichtung mit einem Messelement 111 zur Erfassung der Dejustierung in der Ausrichtung insbesondere Verkippung der optischen Achsen 106 von Sender 105 und Empfänger 103 zueinander vorgesehen sind. Der Sender 105 umfasst eine Lichtquelle und eine Linsenanordnung 113, und der Empfänger 103 weist eine Linsenanordnung 116, ein Messelement 111 und einen Detektor 114 auf. Eine solche Anordnung wird von der Anmelderin zur Gasmessung im Freifeld eingesetzt, wobei das Messelement 111 als in einem vorbestimmten Abstand zum Detektor 114 angeordneter Kreisring ausgebildet ist, auf dem in gleichmäßigen Abstand über den gesamten Umfang eine Anzahl von lichtempfindlichen Sensoren angeordnet ist. Dieser Kreisring wirkt als eine Lochblende, die einen inneren Kernstrahl 109 des von der Lichtquelle des Senders 105 abgestrahlten parallelen Lichtbündels zum Detektor 114 durchlässt und einen äußeren Randstrahl des parallelen Lichtbündels auf den Kreisring selbst und die darauf angeordneten lichtempfindlichen Sensoren auffängt und somit nicht zum Detektor 114 gelangen lässt. Im justiertem Zustand des Empfängers 103 erfassen alle lichtempfindlichen Sensoren die gleiche Intensität. Bei einer Dejustierung des Empfängers 103 erfassen einige lichtempfindliche Sensoren jedoch kein Licht oder weniger Licht, wobei aus dieser Verteilung eine Information über die Dejustierung gewonnen werden kann. Allerdings ergibt sich aus dieser Anordnung des Messelementes 111 ein zusätzlicher Lichtverlust, da ein Teil des vom Sender 105 abgestrahlten Lichtes, das für die Justierung von Sender und Empfänger benötigt wird, den Detektor 114 nicht erreicht und somit für die Messung am Detektor 114 nicht mehr beitragen kann.

Darüber hinaus ergibt sich grundsätzlich das Problem, dass bei den verwendeten Detektoren die Sensitivität als Funktion des Ortes auf der Detektorfläche nicht konstant ist, sondern zum Rand hin zunimmt. Wenn also der auf den Detektor auftreffende Strahlfleck kleiner als die Detektorfläche ist oder die Detektorfläche nicht homogen ausgeleuchtet ist, ändert sich allein bei einer Verschiebung des Strahlflecks die gemessene Intensität. Da aber das Verhältnis der am Analysedetektor und am Referenzdetektor gemessenen Intensitäten als Maß für eine Gaskonzentration verwendet wird, kann schon eine geringe Veränderung der Justierung des Senders oder des Empfängers zu einem signifikant verändertem Messsignal führen.

Ein weiteres Problem ist, dass der Durchlassbereich bzw. die Kantenwellenlänge bei den in Open-Path-Systemen verwendeten Bandpassfiltern und Kantenfiltern eine Funktion des Einfallswinkels ist. Dabei ist die Änderung der jeweiligen Grenzwellenlängen dann besonders groß, wenn der Einfallswinkel auf den Filter im Bereich von 45° liegt. Da eine solche Anordnung in Gasdetektorsystemen häufig verwendet wird, um den Teil, der von dem Filter nicht durchgelassen wird, auf eine andere Messanordnung zu reflektieren, kann eine Fehljustierung auch hier schnell dazu führen, dass sich der spektrale Durchlassbereich bzw. die Kantenwellenlänge verschieben und dadurch ebenfalls ein geändertes Messergebnis produziert wird.

Wenn nach einer groben Justierung der Sender und der Empfänger so zueinander ausgerichtet sind, dass die Detektoren im Empfänger den Analyselichtstrahl "sehen", gibt es noch zwei Möglichkeiten, in welcher Weise Sender und Empfänger fehlausgerichtet sind. Zum einen kann der Sender gegenüber der Verbindungslinie zwischen Sender und Empfänger leicht verkippt sein, und zum anderen ist es auch denkbar, dass der Empfänger gegenüber dieser Verbindungslinie gekippt ist. Daher ist es wünschenswert, wenn Sender und Empfänger derart ausgestaltet sind, dass es ohne Weiteres möglich ist, zu identifizieren, ob eine oder beide der zuvor genannten Fälle vorliegen.

Hierbei soll das Gasdetektorsystem insbesondere so ausgestaltet sein, dass es sich in einfacher Weise, möglichst ohne Zuhilfenahme komplizierter Optiken, ausrichten lässt.

Ausgehend vom Stand der Technik ist es daher die Aufgabe, ein Gasdetektorsystem bereitzustellen, bei dem Fehlausrichtungen innerhalb eines Toleranzbereichs einen nur sehr geringen Einfluss auf die vom Analysedetektor erfasste Intensität haben.

Erfindungsgemäß wird diese Aufgabe gelöst durch ein Gasdetektorsystem umfassend einen Sender mit einer Lichtquelle, von der ein Analyselichtstrahl emittiert wird, und mit einer Sender-Linsenordnung, die ausgestaltet ist, den Analyselichtstrahl entlang einer Emissionsrichtung zu fokussieren, und einen Empfänger mit einer Empfänger-Linsenanordnung, die einen Empfänger-Brennpunkt und eine Empfängerachse definiert, mit einem ersten Lichtmischstab, der ein erstes Eintrittsende und ein erstes Austrittsende aufweist und eine sich von dem ersten Eintrittsende zu dem ersten Austrittsende verlaufende erste Stabachse definiert, wobei das erste Eintrittsende zu der Empfänger-Linsenanordnung weist, mit einem Analysedetektor und einem Referenzdetektor, wobei der Analysedetektor auf der von der Empfänger-Linsenanordnung weg weisenden Seite des ersten Austrittsendes angeordnet ist, mit einem Analysefilter, der von der Empfänger-Linsenanordnung aus gesehen vor dem Analysedetektor angeordnet ist, und mit einem Referenzfilter der von der Empfänger-Linsenanordnung gesehen vor dem Referenzdetektor angeordnet ist.

Unter einem Lichtmischstab im Sinne der vorliegenden Erfindung wird ein sich entlang einer Stabachse erstreckender, zu dieser eine Symmetrie aufweisender Körper oder Hohlkörper verstanden, dessen Außenwandung derart ausgestaltet ist, dass durch das Eintrittsende ins Innere des Körpers oder Hohlkörpers eintreffende Strahlung an der Umfangswandung reflektiert und damit im Inneren des Körpers gehalten wird. Dies führt dazu, dass am Eintrittsende eintretende Strahlung den Körper oder Hohlkörper am Austrittsende als Strahlenbündel mit einer über die Querschnittsfläche des Strahlenbündels homogenen Intensität verlässt, wenn der Brennpunkt der am Eintrittsende eintretenden Strahlung in der Ebene des Eintrittsendes oder kurz dahinter im Inneren des Körpers oder Hohlkörpers liegt. Derartige Lichtmischstäbe sind aus Projektoren bekannt.

Durch den Einsatz eines Lichtmischstabs im Empfänger wird ermöglicht, die auf die Empfänger-Linsenanordnung einfallende, vom Sender kommende Strahlung derart zu bündeln, dass der auf den Analysedetektor auftreffende Strahlfleck homogen ausgeleuchtet ist. Sofern dieser Strahlfleck größer als die Detektorfläche ist, führt eine geringe Dejustierung des Empfängers, bei der der Strahlfleck gegenüber der optimalen Position, in der der Detektor im Zentrum des Strahlflecks liegt, nicht zu einer Änderung des vom Detektor abgegebenen Signals. Dadurch wird der Toleranzbereich, innerhalb dessen der Empfänger dejustiert sein kann, gegenüber herkömmlichen Systemen vergrößert.

Hierbei ist es bevorzugt, wenn die Empfängerlinsen-Anordnung eine Feldlinse aufweist, die vor dem Eintrittsende angeordnet ist. Dies führt dazu, dass das in den Lichtmischstab einfallende Licht so kollimiert wird, dass der nach dem Lichtmischstab gebildete Strahlfleck sehr homogen ausgeleuchtet ist.

Vorzugsweise liegt der Brennpunkt innerhalb des ersten Lichtmischstabs, was mit dem Vorteil verbunden ist, dass es nicht dazu kommen kann, dass Verschmutzungen in Bereichen auftreten können, in denen der Strahl stark kollimiert ist. Dies hätte zur Folge, dass große Intensitätsverluste aufträten. Diese Gefahr bestünde insbesondere dann, wenn der Brennpunkt auf einer Oberfläche des Lichtmischstabes an dem Eintrittsende läge. Außerdem wird bei einem solchen bevorzugten Aufbau Baulänge eingespart.

Gemäß einer bevorzugten Ausführungsform verläuft die erste Strahlachse entlang der Empfängerachse, wobei das erste Austrittsende eine Prismenanordnung zum Aufspalten eines den ersten Lichtmischstab verlassenden Strahls in einen ersten Strahl und einen zweiten Strahl aufweist, wobei sich der erste Strahl entlang einer ersten Richtung ausbreitet und der zweite Strahl entlang einer zweiten Richtung und die erste und zweite Richtung unter einem Winkel zu der ersten Stabachse verlaufen, wobei der Analysedetektor entlang der ersten Richtung angeordnet ist, wobei der Referenzdetektor entlang der zweiten Richtung angeordnet ist, wobei zwischen dem Analysedetektor und dem ersten Austrittsende der Analysefilter angeordnet ist und wobei zwischen dem Referenzdetektor und dem ersten Austrittsende der Referenzfilter angeordnet ist.

Bei einer derartigen Anordnung ist ein Lichtmischstab ausreichend, wobei die Prismenanordnung dann dazu dient, den homogen ausgeleuchteten Strahlfleck sowohl auf den Analysedetektor als auch auf den Referenzdetektor abzubilden. Hierbei ist es nicht nötig, den in den Empfänger einfallenden Strahl mit Hilfe eines Kantenfilters aufzuspalten, wobei der Analysestrahl und der Referenzstrahl dann jeweils entlang separater Pfade ggf. noch durch getrennte Lichtmischstäbe laufen.

Bei dieser bevorzugten Ausführungsform sind vor den jeweiligen Detektoren entsprechende Bandpassfilter angeordnet, deren Durchlassbereiche jedoch voneinander abweichen, um einerseits den Wellenlängenbereich mit den fraglichen Absorptionsbanden und andererseits einen Ausschnitt aus dem Strahl ohne diese Bande zu erhalten und zu erfassen.

Schließlich kann eine Empfängerlichtquelle auf der von der Empfänger-Linsenanordnung weg weisenden Seite des Austrittsendes auf der Empfängerachse angeordnet sein. Licht, das von dieser Empfängerlichtquelle ausgesandt wird, trifft dann auf den Sender. Wenn dieser einen zwischen der Sender-Linsenanordnung und der Lichtquelle angeordneten Filter aufweist, der entgegen der Emissionsrichtung auf die Sender-Linsenanordnung fallendes Licht auf eine in dem Sender vorgesehene erste positionssensitive Detektionseinrichtung reflektiert, kann das Signal dieser Detektionseinrichtung dazu verwendet werden, zu bestimmen, in welcher Weise der Sender gegenüber der Verbindungslinie zwischen Sender und Empfänger verkippt ist. Dadurch kann das Ausrichten des Senders stark vereinfacht werden.

Des Weiteren ist es bevorzugt, wenn an der sich zwischen dem Eintrittsende und dem Austrittsende des ersten Lichtmischstabs erstreckenden Umfangswandung in Umfangsrichtung beabstandet lichtempfindliche Sensoren angeordnet sind, deren lichtempfindliche Bereiche zum Inneren des Lichtmischstabs weisen. Wenn der Brennpunkt des auf das Eintrittsende des ersten Lichtmischstabs auftreffenden Strahlenbündels nicht auf der Stabachse liegt, sondern seitlich dazu versetzt ist, trifft mehr Licht auf dieser Seite auf den entsprechenden lichtempfindlichen Sensor, so dass diese zuvor beschriebene Anordnung ermöglicht, die Fehlausrichtung des Empfängers anhand der Signale der Sensoren zu bestimmen. Somit kann in einfacher Weise auch eine ggf. vorhandene Verkippung des Empfängers gegenüber der Verbindungslinie zwischen Sender und Empfänger ohne Zuhilfenahme von optischen Instrumenten bestimmt werden.

Hierbei ist besonders bevorzugt, wenn der erste Lichtmischstab einen senkrecht zur ersten Stabachse gesehen polygonalen Querschnitt aufweist. Bei einer solchen, im Vergleich zu einem kreisförmigen Querschnitt geringeren Symmetrie ergibt sich eine größere Homogenisierung des Strahls, und gleichzeitig lassen sich in einfacher Weise die Sensoren anbringen.

In einer alternativen Ausführungsform weist der Empfänger einen zweiten Lichtmischstab auf, der ein zweites Eintrittsende und ein zweites Austrittsende aufweist und eine sich von dem zweiten Eintrittsende zu dem zweiten Austrittsende verlaufende zweite Stabachse definiert, wobei das zweite Eintrittsende in Richtung eines in den Empfänger fallenden Strahls gesehen näher zu der Empfänger-Linsenanordnung angeordnet ist, wobei die erste und die zweite Stabachse unter einem Winkel zueinander verlaufen, wobei der Referenzdetektor auf der in Richtung eines in den Empfänger fallenden Strahls gesehen von der Empfänger-Linsenanordnung weg weisenden Seite des zweiten Austrittsendes angeordnet ist, wobei der Analysefilter zwischen dem ersten Eintrittsende und der Empfänger-Linsenanordnung angeordnet ist, wobei der Analysefilter derart ausgerichtet ist, dass entlang der Empfängerachse auf den Analysefilter fallendes und nicht-durchgelassenes Licht entlang der zweiten Stabachse auf das zweite Eintrittsende reflektiert wird und wobei der Referenzfilter zwischen der Empfänger-Linsenanordnung und dem Analysefilter angeordnet ist.

Eine derartige Anordnung ermöglicht, für den Analysedetektor und den Referenzdetektor gemeinsame Referenz- und Analysefilter zu verwenden.

Außerdem können in diesem Fall die Lichtmischstäbe einfacher ausgestaltet sein und benötigen keine kostenspielige Prismenanordnung am Austrittsende. Dadurch ist auch die in den Detektoren erfasste Intensität grundsätzlich höher, was wiederum die Nachweiswahrscheinlichkeit der zu detektierenden Gaskomponente erhöht.

In einer bevorzugten Ausführungsform ist die erste Stabachse entlang der Empfängerachse ausgerichtet, während die zweite Stabachse unter einem Winkel, vorzugsweise senkrecht, zu der Empfängerachse verläuft. In diesem Fall kann die Anordnung in einfacher Weise mit nur wenigen optischen Elementen realisiert werden. Insbesondere können dann die weiteren optischen Elemente des Empfängers bei der Montage leicht justiert werden.

Bei einer derartigen Anordnung ist es auch möglich, dass der Referenzfilter derart ausgerichtet ist, dass entlang der Empfängerachse auf den Referenzfilter fallendes und nicht-durchgelassenes Licht auf eine in dem Empfänger vorgesehene zweite positionssensitive Detektionseinrichtung reflektiert wird. In diesem Fall kann das Signal in der positionssensitiven Detektionseinrichtung dazu dienen, zu bestimmen, ob der Empfänger korrekt zu der Verbindungslinie zwischen Sender und Empfänger ausgerichtet ist.

Des Weiteren ist es bei der zuvor beschriebenen bevorzugten Ausführungsform vorteilhaft, wenn die optischen Weglängen zwischen der Empfänger-Linsenanordnung und dem ersten Lichtmischstab und zwischen der Empfänger-Linsenanordnung und dem zweiten Lichtmischstab derart bemessen sind, dass der Brennpunkt der Linsenanordnung des Empfängers innerhalb des ersten und des zweiten Lichtmischstabs liegt. Dies ist wiederum mit dem Vorteil verbunden, dass nicht bereits geringe Verschmutzungen im Inneren des Empfängers zu erheblichen Intensitätseinbußen bei den Detektoren führen können.

Des Weiteren ist es vorteilhaft, wenn der Referenzfilter als Bandpassfilter ausgebildet ist und der Analysefilter als Kantenfilter und die untere Grenzwellenlänge des Bandpassfilters geringer ist als die Kantenwellenlänge des Kantenfilters. Auf diese Weise kann zunächst durch den Bandpassfilter nur das Licht aus dem von der Lichtquelle ausgesandten Licht ausgewählt werden, das für die Messung relevante Spektrum enthält. Anschließend dient der Kantenfilter dazu, nur noch das Licht zu dem Analysedetektor durchzulassen, das die fraglichen Absorptionslinien enthält.

Damit der auf die Detektoren auftreffende Strahlfleck nicht zu groß ist, so dass die gesamte Intensität auf die Detektoren auftrifft, das System aber dennoch hinreichend fehlertolerant ist, ist es bevorzugt, wenn die Lichtmischstäbe konisch, sich zum Austrittsende hin verjüngend, ausgebildet sind.

Schließlich ist es besonders bevorzugt, wenn die verwendeten positionssensitiven Detektionseinrichtungen als CCD-, CMOS- oder Quadrantendetektor ausgebildet sind, da diese kostengünstig sind und zuverlässig ausgewertet werden können.

Im Folgenden wird die vorliegende Erfindung anhand einer Zeichnung erläutert, wobei
- Fig. 1: ein System gemäß dem Stand der Technik zeigt,
- Fig. 2: den Sender eines ersten Ausführungsbeispiels der Erfindung zeigt,
- Fig. 3: den Empfänger des ersten Ausführungsbeispiels zeigt
- Fig. 4: ein Teil des Empfängers aus Fig. 3 in vergrößerter Darstellung zeigt und
- Fig. 5: den Empfänger des zweiten Ausführungsbeispiels zeigt.

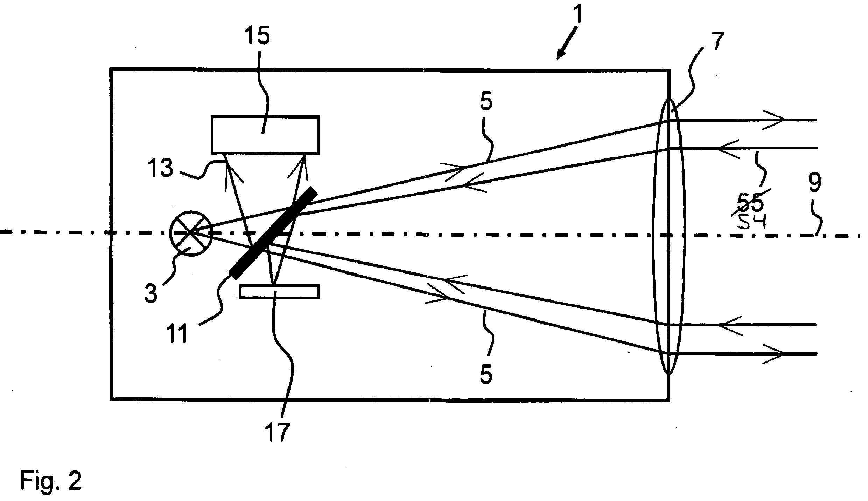

Wie aus Figur 2 zu erkennen ist, weist der Sender 1 eines ersten Ausführungsbeispiels eines erfindungsgemäßen Gasdetektorsystems eine Lichtquelle 3 auf, von der ein Analyselichtstrahl 5 emittiert wird. Des Weiteren ist in dem Sender 1 eine im vorliegenden Fall nur eine Linse umfassende Sender-Linsenanordnung 7 vorgesehen, mit der das von der Lichtquelle 3 emittierte Licht entlang einer Emissionsrichtung 9 kollimiert wird.

Außerdem ist in dem Sender 1 ein Farbteiler 11 derart angeordnet, dass von der Lichtquelle 3 emittiertes Licht, bevor es zu der Sender-Linsenanordnung 7 gelangt, auf den Farbteiler 11 fällt. Dieser wirkt derart, dass Infrarotlicht ungehindert durch den Farbteiler 11 hindurch tritt und somit den Analyselichtstrahl 5 bildet, während sichtbares Licht 13 vom Farbteiler 11 reflektiert und in eine Strahlfalle 15 geworfen wird.

Die von der Lichtquelle 3 abgewandte und zu der Sender-Linsenanordnung 7 weisende Seite des Farbteilers 11 ist derart ausgebildet, dass auf diese Seite fallendes sichtbares Licht reflektiert wird. Der Farbteiler 11 ist dabei so zu der Sender-Linsenanordnung 7 ausgerichtet, dass entlang der Emissionsrichtung 9 auf die Sender-Linsenanordnung 7 einfallendes Licht von dem Farbteiler 11 auf einen ebenfalls in dem Sender 1 angeordneten, positionssensitiven Sender- Detektor 17 geworfen wird, der eine erste positionssensitive Detektionseinrichtung bildet.

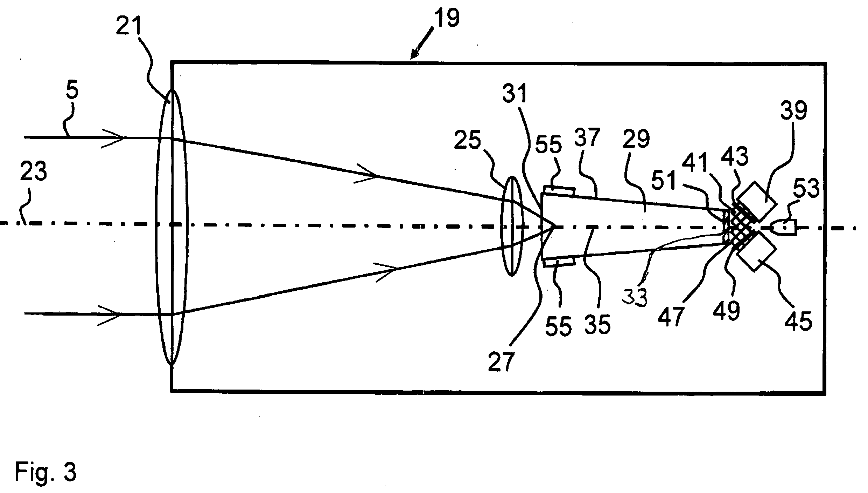

Wie aus Figur 3 hervorgeht, weist der Empfänger 19 des ersten Ausführungsbeispiels eine Empfänger-Linsenanordnung 21 auf, die im vorliegenden Ausführungsbeispiel ebenfalls aus einer einzelnen Linse gebildet ist und die eine Empfängerachse 23 definiert. Ebenfalls auf der Empfängerachse 23 angeordnet ist eine Feldlinse 25. Diese dient dazu, die bereits von der Empfänger-Linsenanordnung 21 fokussierten Strahlen weiter zusammenzuführen. Außerdem wird durch die Empfänger-Linsenanordnung 21 ein Empfänger-Brennpunkt 27 definiert, der auf der Empfängerachse 23 liegt.

Des Weiteren ist in dem Empfänger 19 auf der Empfängerachse 23 ein erster Lichtmischstab 29 vorgesehen, der ein erstes Eintrittsende 31 sowie ein erstes Austrittsende 33 aufweist. Zwischen dem ersten Eintrittsende 31 und dem ersten Austrittsende 33 erstreckt sich die durch den Lichtmischstab 29 definierte erste Stabachse 35, die im vorliegenden Ausführungsbeispiel zu der Empfängerachse 23 ausgerichtet ist. Wie ferner aus Figur 3 zu erkennen ist, ist der erste Lichtmischstab 29 derart angeordnet, dass der Empfänger-Brennpunkt 27 innerhalb des ersten Lichtmischstabs 29 liegt.

Unter einem Lichtmischstab 29 im Sinne der vorliegenden Erfindung wird ein sich entlang der Stabachse 35 erstreckender und zu dieser eine Symmetrie aufweisender Körper oder Hohlkörper verstanden, der transparent ist und dessen Umfangswandung 37 derart ausgestaltet ist, dass ins Innere des Lichtmischstabs 29 eintreffendes Licht an der Umfangswandung 37 reflektiert und damit im Inneren des Lichtmischstabs 29 gehalten wird. Dies führt dazu, dass am Eintrittsende 31 eintretende Strahlung den Lichtmischstab 29 am Austrittsende 33 als Strahlenbündel mit homogener Intensität über den Querschnitt verlässt.

In bevorzugter Weise weist der Lichtmischstab 29 einen senkrecht zur Stabachse 35 gesehen polygonalen Querschnitt auf, wobei der Lichtmischstab sich zum Austrittsende 33 hin verjüngend konisch ausgebildet sein kann. Bei einer solchen, im Vergleich zu einem kreisförmigen Querschnitt geringeren Symmetrie ergibt sich eine größere Homogenisierung des Strahls.

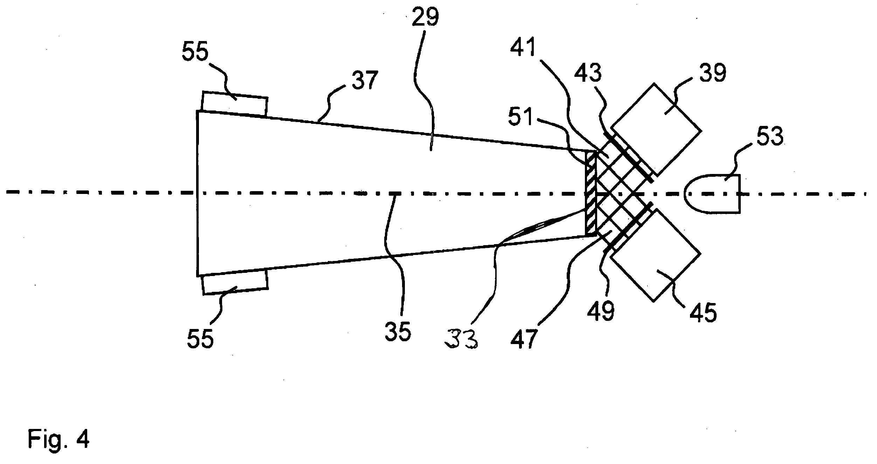

Wie aus den Figuren 3 und 4 hervorgeht, ist auf der von der Empfänger-Linsenanordnung 21 weg weisenden Seite des ersten Austrittsendes 33 des Lichtmischstabs 29 ein Analysedetektor 39 angeordnet, wobei der Analysedetektor 39 entlang einer ersten Richtung angebracht ist, entlang derer sich ein erster Strahl 41, der aus dem ersten Austrittsende 33 des Lichtmischstabs 29 austritt, ausbreitet. Schließlich ist zwischen dem Analysedetektor 39 und dem ersten Austrittsende 33 ein Analysefilter 43 vorgesehen.

Ebenfalls auf der von der Empfängerlinsenanordnung 21 weg weisenden Seite des ersten Austrittsendes 33 ist ein Referenzdetektor 45 vorgesehen, wobei dieser entlang einer zweiten Richtung angeordnet ist, entlang derer ein zweiter Strahl 47 aus dem ersten Austrittsende 33 des Lichtmischstabs 29 austritt. Zwischen dem Referenzdetektor 45 und dem ersten Austrittsende 33 ist schließlich noch ein Referenzfilter 49 vorgesehen. Sowohl der erste Strahl 41 als auch der zweite Strahl 47 bzw. die erste und die zweite Richtung verlaufen gegenüber der ersten Stabachse 35 und der Empfängerachse 23 in einem Winkel.

Damit sich der erste und zweite Strahl 41, 47 unter jeweils einem Winkel gegenüber der Empfängerachse 23 aus dem ersten Austrittsende 33 heraus ausbreiten, ist eine Prismenanordnung 51 zum Aufspalten des den ersten Lichtmischstab 29 verlassenden Strahls vorgesehen, die diesen in den ersten und zweiten Strahl 41, 47 aufspaltet. Insbesondere kann die Prismenanordnung derart ausgestaltet sein, dass sie eine Vielzahl von sich senkrecht zur Zeichenebene erstreckende Einzelprismen aufweist, die parallel zueinander angeordnet sind.

Sowohl der Analysefilter 43 als auch der Referenzfilter 49 sind als Bandpassfilter ausgebildet, wobei deren Durchlassbereiche allerdings voneinander abweichen und einander nicht überlappen.

Schließlich ist aus den Figuren 3 und 4 zu erkennen, dass der Empfänger 19 eine auf der Empfängerachse 23 liegende Empfänger-Lichtquelle 53 aufweist, die als Leuchtdiode ausgebildet sein kann und Licht entlang der Empfängerachse 23 in Richtung der Empfänger-Linsenanordnung 21 emittiert.

Des Weiteren ist den Figuren 3 und 4 zu entnehmen, dass in Umfangsrichtung beabstandet lichtempfindliche Sensoren 55 an der Umfangswandung 37 des Lichtmischstabs 29 angeordnet sind, wobei die Sensoren 55 zum Inneren des Lichtmischstabs 29 weisen.

Das in den Figuren 2 bis 4 dargestellte und zuvor beschriebene erste Ausführungsbeispiel eines erfindungsgemäßen Gasdetektorsystems arbeitet wie folgt.

Von der Lichtquelle 3 wird ein Analyselichtstrahl 5 ausgesandt, der zunächst den Farbteiler 11 durchläuft, wobei sichtbares Licht 13 in der Strahlfalle 15 aufgefangen wird, ohne dass es sich weiter in dem Empfänger 1 ausbreiten kann. Der Analyselichtstrahl 5 wird durch die Sender-Linsenanordnung 7 zu einem parallelen Strahlenbündel fokussiert, das dann nach Durchlaufen der Messstrecke auf die Empfänger-Linsenanordnung 21 fällt, von der es, sofern Sender 1 und Empfänger 19 so zueinander ausgerichtet sind, dass Emissionsrichtung 9 und Empfängerachse 23 zusammenfallen, in den Empfängerbrennpunkt 27 fokussiert wird, der in dem ersten Lichtmischstab 29 angeordnet ist. Durch den ersten Lichtmischstab 29 wird der einfallende Analyselichtstrahl 5 durch mehrmalige Reflexion an der Umfangswandung 37 homogenisiert, so dass der an dem ersten Austrittsende 33 austretende Lichtstrahl über das erste Austrittsende 33 gesehen homogen ist, also über die Fläche des ersten Austrittsendes gesehen eine nahezu gleichmäßige Intensität aufweist.

Die am ersten Austrittsende 33 vorgesehene Prismenanordnung 51 spaltet das austretende Licht in einen ersten Strahl 41, der zunächst durch den Analysefilter 43 fällt und dann in den Analysedetektor 39 trifft, und einen zweiten Strahl 47 auf, der durch den Referenzfilter 49 in den Referenzdetektor 45 fällt. Der Durchlassbereich des Analysefilters 43 ist so gewählt, dass der Bereich des Frequenzspektrums, in dem die Absorptionsbanden der entlang der Messstrecke zu detektierenden Gaskomponente liegen, durchgelassen wird. Der Durchlassbereich des Referenzfilters 49 überlappt nicht mit dem Durchlassbereich des Analysefilters 43, so dass der Referenzdetektor 45 ein von der fraglichen Gaskonzentration unabhängiges Signal ausgibt, während von dem Analysedetektor 39 ein Signal ausgegeben wird, das abhängig ist von der Konzentration der fraglichen Gaskomponente entlang der Messstrecke. Aus dem Verhältnis der beiden Signale kann dann auf die absolute Konzentration der Gaskomponente geschlossen werden.

Sofern der von den ersten und zweiten Strahlen 41, 47 auf den Detektoren 39, 45 erzeugte Strahlfleck größer als die jeweilige Detektorfläche ist, führt eine geringe Dejustierung des Empfängers 19, bei der der Strahlfleck gegenüber der optimalen Position, in der die Detektoren 39, 45 im Zentrum des Strahlflecks liegen, nicht zu einer Änderung der von Detektoren 39, 45 abgegebenen Signalen, da der Strahlfleck jeweils aufgrund des Lichtmischstabs 29 homogen ausgeleuchtet ist. Dadurch wird der Toleranzbereich, innerhalb dessen der Empfänger 19 dejustiert sein kann, gegenüber herkömmlichen Systemen vergrößert. Ferner ist bei dem zuvor beschriebenen Aufbau des Empfängers 19 ein einziger Lichtmischstab 29 ausreichend, wobei die Prismenanordnung 51 dann dazu dient, den homogen ausgeleuchteten Strahlfleck sowohl auf den Analysedetektor 39 als auch auf den Referenzdetektor 45 abzubilden.

Mit Hilfe der an dem ersten Lichtmischstab 29 angebrachten Sensoren 55 kann festgestellt werden, ob die Empfängerachse 23 gegenüber der Verbindungslinie zwischen Sender 1 und Empfänger 19 verkippt ist. Dabei werden die von den Sensoren 55 erfassten Intensitäten verglichen, und wenn eine der erfassten Intensitäten höher ist als die übrigen, deutet dies darauf hin, dass die Empfängerachse 23 in Richtung dieses Sensors 55 gegenüber der Verbindungslinie verkippt ist. Dann kann der Empfänger 19 entsprechend rejustiert werden.

Schließlich kann mit Hilfe der Empfängerlichtquelle 53 ein Justierstrahl 54 erzeugt werden, der zunächst von dem Lichtmischstab 29 homogenisiert und anschließend von der Empfänger-Linsenanordnung 21 in ein paralleles Strahlenbündel 54 konvertiert wird, das durch die Sender-Linsenanordnung 7 in den Sender 1 fällt und von dem Farbteiler 11 auf den positionssensitiven Sender- Detektor 17 reflektiert wird. Anhand der von diesem Detektor 17 erfassten Position kann festgestellt werden, ob die Emissionsrichtung 9 tatsächlich mit der Verbindungslinie zwischen Sender 1 und Empfänger 19 zusammenfällt oder gegenüber dieser verkippt ist.

Somit ist mit diesem ersten Ausführungsbeispiel eines Gasdetektorsystems zum einen aufgrund des Lichtmischstabs 29 gewährleistet, dass ein homogener Strahl am Austrittsende 33 des Lichtmischstabs 29 erzeugt wird, der dann auf die Detektoren 39, 45 fällt, wobei eine geringe Fehljustierung keinen Einfluss auf das von den Detektoren 39, 45 ausgegebene Signal hat, da der Strahl homogen ist. Des Weiteren erlauben die Sensoren 55 sowie die Empfängerlichtquelle 53 zusammen mit dem Sender- Detektor 17 eine einfache und ggfs. automatisierbare Justage des Gasdetektorsystems.

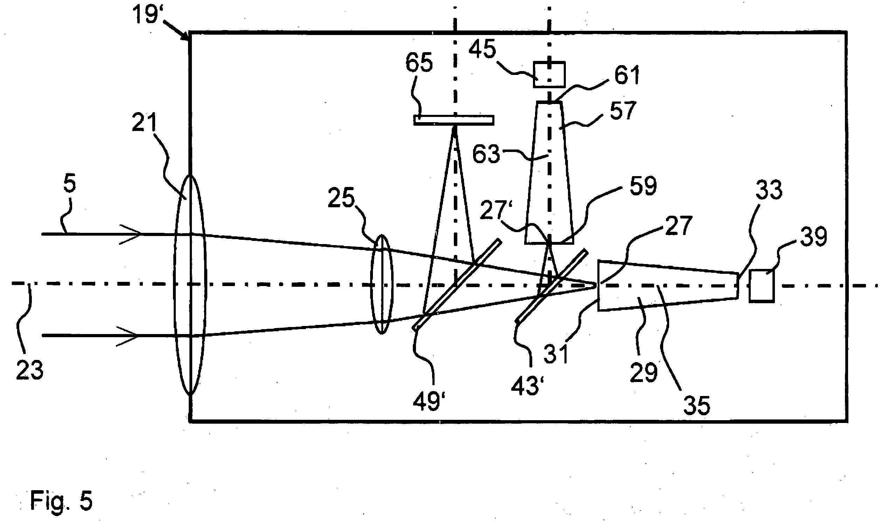

In Figur 5 ist ein zweites Ausführungsbeispiel eines Empfängers 19' dargstellt, wobei dieser Empfänger 19' ebenfalls eine Empfänger-Linsenanordnung 21 aufweist, die eine Empfängerachse 23 definiert und zusammen mit einer Feldlinse 25 den einfallenden Analysestrahl 5 in einen Empfänger-Brennpunkt 27, 27' fokussiert. Ein Empfängerbrennpunkt 27 liegt dabei in einem ersten Lichtmischstab 29, der wiederum ein erstes Eintrittsende 31 und ein erstes Austrittsende 33 sowie eine sich dazwischen erstreckende erste Stabachse 35 aufweist. Des Weiteren ist in dem Empfänger 19' auf der von der Empfängerlinsenanordnung 21 weg weisenden Seite des ersten Austrittsendes 33 ein Analysedetektor 39 vorgesehen.

Neben dem ersten Lichtmischstab 29 ist bei diesem Ausführungsbeispiel noch ein zweiter Lichtmischstab 57 vorgesehen, der in analoger Weise ein zweites Eintrittsende 59 und ein zweites Austrittsende 61, sowie eine sich dazwischen erstreckende zweite Stabachse 63 aufweist. Dabei sind die Lichtmischstäbe 57 ähnlich dem ersten Lichtmischstab 29 des ersten Ausführungsbeispiels eines Empfänger 19 ausgebildet, d.h. haben einen polygonalen Querschnitt und verjüngen sich zu den Austrittsenden 33, 61 hin konisch.

Wie weiter zu erkennen ist, sind im vorliegenden Ausführungsbeispiel die erste Stabachse 35 und die zweite Stabachse 63 senkrecht zueinander angeordnet, und die erste Stabachse 35 erstreckt sich entlang der Empfängerachse 23. Es ist aber auch denkbar, dass sich die Stabachsen 35, 63 unter einem Winkel erstrecken, der von 90° abweicht.

Im Schnittpunkt der Verlängerung der Stabachsen 35, 63 über das jeweilige Eintrittsende 31, 59 hinaus ist ein Analysefilter 43' angeordnet, durch den der Teil des Analyselichtstrahls 5 hindurch tritt, der die Absorptionbanden der zu untersuchenden Gaskomponente enthält, während der Analysefilter 43' den übrigen Teil des Analyselichtstrahls 5 in Richtung des zweiten Lichtmischstabes 57 reflektiert. Insbesondere kann der Analysefilter 43' als Kantenfilter ausgebildet sein, dessen Kantenwellenlänge, also diejenige Wellenlänge, oberhalb derer die Transmission ansteigt, unmittelbar vor den Absorptionsbanden liegt.

Auf der in Richtung eines in den Empfänger 19' einfallenden Strahls gesehen von der Empfängerlinsenanordnung 21 weg weisenden Seite des zweiten Austrittsendes 61 des zweiten Lichtmischstabs 57 ist ein Referenzdetektor 45 vorgesehen, mit dem das an dem Analysefilter 43' reflektierte und in dem zweiten Lichtmischstab 57 homogenisierte Licht erfasst wird.

Auch bei diesem Ausführungsbeispiel sind die Lichtmischstäbe 29, 57 derart angeordnet, dass die optischen Weglängen zwischen der Empfänger-Linsenanordnung 21 und dem ersten Lichtmischstab 29 und zwischen der Empfänger-Linsenanordnung 21 und dem zweiten Lichtmischstab 57 jeweils so bemessen sind, dass der Brennpunkt 27, 27' innerhalb des ersten und des zweiten Lichtmischstabs 29, 57 liegt.

Schließlich weist der Empfänger 19' noch einen Referenzfilter 49' auf, der zwischen der Empfänger-Linsenanordnung 21 und dem Analysefilter 43' angeordnet ist. Der Referenzfilter 49' ist als Bandpassfilter ausgebildet und lässt denjenigen Wellenlängenbereich passieren, in dem die fraglichen Absoprtionsbanden liegen, sowie einen Bereich davor. Der übrige Teil des Lichts wird reflektiert. Dabei ist der Referenzfilter 49' derart ausgerichtet, dass entlang der Empfängerachse 23 darauf fallendes und nicht durchgelassenes Licht auf eine zweite positionssensitive Detektionseinrichtung, nämlich einen Empfänger- Detektor 65, fällt.

Dieses zweite Ausführungsbeispiel eines Empfänger 19' arbeitet nun wie folgt. Der einfallende Analysestrahl 5 wird von der Empfänger-Linsenanordnung 21 durch den teilweise reflektierend wirkenden Analysefilter 43' sowohl in den ersten Lichtmischstab 29 als auch in den zweiten Lichtmischstab 57 geworfen, wobei in den ersten Lichtmischstab 29 dasjenige Licht mit den fraglichen Absorptionsbanden trifft und darin so homogenisiert wird, dass ein homogener Strahlfleck auf den Analysedetektor 39 trifft. In ähnlicher Weise wird der Teil, der keine Absorptionsbande enthält, in den Referenzdetektor 45 in homogener Weise geworfen. Bei diesem Aufbau wird keine kostenaufwändige Prismenanordnung am Austrittsende eines Lichtmischstabes benötigt, und die in den Detektoren 39, 45 erfasste Intensität ist grundsätzlich höher, was wiederum die Nachweiswahrscheinlichkeit der zu detektierenden Gaskomponente erhöht.

Aus der Verwendung von Lichtmischstäben 29, 57 ergibt sich auch bei diesem Ausführungsbeispiel, dass eine leichte Dejustage des Empfängers 19' dann nicht zu einer Änderung des Messergebnisses führt, wenn der aus den Lichtmischstäben 29, 57 am Austrittsende 33, 61 austretende Strahl jeweils eine Fläche hat, die größer ist als der sensitive Bereich der jeweiligen Detektoren 39, 45 und bei der Dejustage der Detektor 39, 45 trotzdem vollständig bestrahlt wird.

Darüber hinaus kann das am Referenzfilter 49' reflektierte Licht dazu benutzt werden, mit Hilfe des Empfänger- Detektors 65 zu bestimmen, ob die Empfängerachse 23 tatsächlich mit der Verbindungslinie zwischen Sender 1 und Empfänger 19' zusammenfällt oder etwas gegenüber dieser verkippt ist.

### Bezugszeichenliste

- 1: Sender
- 3: Lichtquelle
- 5: Analyselichtstrahl
- 7: Sender-Linsenanordnung
- 9: Emissionsrichtung
- 11: Farbteiler
- 13: sichtbares Licht
- 15: Strahlfalle
- 17: Sender- Detektor
- 19, 19': Empfänger
- 21: Empfänger-Linsenanordnung
- 23: Empfängerachse
- 25: Feldlinse
- 27: Empfänger-Brennpunkt
- 29: erster Lichtmischstab
- 31: erstes Eintrittsende
- 33: erstes Austrittsende
- 35: erste Stabachse
- 37: Umfangswandung
- 39: Analysedetektor
- 41: erster Strahl
- 43: Analysefilter
- 45: Referenzdetektor
- 47: zweiter Strahl
- 49: Referenzfilter
- 51: Prismenanordnung
- 53: Empfänger-Lichtquelle
- 54: Justierstrahl, Strahlenbündel
- 55: Sensoren
- 57: zweiter Lichtmischstab
- 59: zweites Eintrittsende
- 61: zweites Austrittsende
- 63: zweite Stabachse
- 65: Empfänger- Detektor

## Patentansprüche

1. Gasdetektorsystem umfassend
einen Sender (1)
mit einer Lichtquelle (3), von der ein Analyselichtstrahl (5) emittiert wird, und
mit einer Sender-Linsenordnung (7), die ausgestaltet ist, den Analyselichtstrahl (5) entlang einer Emissionsrichtung (9) zu kollimieren, und
einen Empfänger (19, 19')
mit einer Empfänger-Linsenanordnung (21), die einen Empfänger-Brennpunkt (27, 27') und eine Empfängerachse (23) definiert,
mit einem Analysedetektor (39) und einem Referenzdetektor (45),
mit einem Analysefilter (43, 43'), der von der Empfänger-Linsenanordnung (21) aus gesehen vor dem Analysedetektor (39) angeordnet ist, und
mit einem Referenzfilter (49, 49') der von der Empfänger-Linsenanordnung (21) gesehen vor dem Referenzdetektor (45) angeordnet ist, **dadurch gekennzeichnet, dass** der Empfänger mit einem ersten Lichtmischstab (29), der ein erstes Eintrittsende (31) und ein erstes Austrittsende (33) aufweist und eine sich von dem ersten Eintrittsende (31) zu dem ersten Austrittsende (33) verlaufende erste Stabachse (35) definiert, wobei das erste Eintrittsende (31) zu der Empfänger-Linsenanordnung (21) weist, ausgerüstet ist und wobei der Analysedetektor (39) auf der von der Empfänger-Linsenanordnung (21) weg. weisenden Seite des ersten Austrittsendes (33) angeordnet ist.

2. Gasdetektorsystem nach Anspruch 1, wobei die Empfängerlinsen-Anordnung eine Feldlinse (25) aufweist, die vor dem Eintrittsende (31) angeordnet ist.

3. Gasdetektorsystem nach Anspruch 1 oder 2, wobei der Brennpunkt (27) innerhalb des ersten Lichtmischstabs (29) liegt.

4. Gasdetektorsystem nach einem der Ansprüche 1 bis 3, wobei die erste Stabachse (35) entlang der Empfängerachse (23) verläuft,
wobei das erste Austrittsende (33) eine Prismenanordnung (51) zum Aufspalten eines den ersten Lichtmischstab (29) verlassenden Strahls in einen ersten Strahl (41) und einen zweiten Strahl (47) aufweist,
wobei sich der erste Strahl (41) entlang einer ersten Richtung ausbreitet und der zweite Strahl (47) entlang einer zweiten Richtung und die erste und zweite Richtung unter einem Winkel zu der ersten Stabachse (35) verlaufen,
wobei der Analysedetektor (39) entlang der ersten Richtung angeordnet ist, wobei der Referenzdetektor (45) entlang der zweiten Richtung angeordnet ist, wobei zwischen. dem Analysedetektor (39) und dem ersten Austrittsende (33) der Analysefilter (43) angeordnet ist und
wobei zwischen dem Referenzdetektor (45) und dem ersten Austrittsende (33) der Referenzfilter (49) angeordnet ist.

5. Gasdetektorsystem nach Anspruch 4, wobei der Analysefilter (43) und der Referenzfilter (49) als Bandpassfilter ausgebildet sind, deren Durchlassbereiche voneinander abweichen.

6. Gasdetektorsystem nach Anspruch 4 oder 5, wobei eine Empfänger-Lichtquelle (53) auf der von der Empfänger-Linsenanordnung (21) weg weisenden Seite des ersten Austrittsendes (33) auf der Empfängerachse (23) angeordnet ist.

7. Gasdetektorsystem nach Anspruch 6, wobei der Sender (1) einen zwischen der Sender-Linsenanordnung (7) und der Lichtquelle (3) angeordneten Filter (11) aufweist, der entgegen der Emissionsrichtung auf die Sender-Linsenanordnung (7) fallendes Licht auf eine in dem Sender (1) vorgesehene erste positionssensitive Detektionseinrichtung (17) reflektiert.

8. Gasdetektorsystem nach einem der Ansprüche 1 bis 7, wobei der erste Lichtmischstab (29) eine sich zwischen dem ersten Eintrittsende (31) und dem ersten Austrittsende (33) erstreckende erste Umfangswandung 37) aufweist,
wobei in Umfangsrichtung beabstandet lichtempfindliche Sensoren (55), vorzugsweise Dioden; auf der ersten Umfangswandung (37) angeordnet sind und
wobei der lichtempfindliche Bereich der Sensoren (55) zum Inneren des ersten Lichtmischstabs (29) weist.

9. Gasdetektorsystem nach Anspruch 8, wobei der erste Lichtmischstab (29) einen senkrecht zur ersten Stabachse (35) gesehen polygonalen Querschnitt aufweist.

10. Gasdetektorsystem nach Anspruch 1 oder 2, wobei der Empfänger (19') einen zweiten Lichtmischstab (57), der ein zweites Eintrittsende (59) und ein zweites Austrittsende (61) aufweist und eine sich von dem zweiten Eintrittsende (59) zu dem zweiten Austrittsende (61) verlaufende zweite Stabachse (63) definiert, wobei das zweite Eintrittsende (59) in Richtung eines in den Empfänger (19') fallenden Strahls gesehen näher zu der Empfänger-Linsenanordnung (21) angeordnet ist,
wobei die erste und die zweite Stabachse (35, 63) unter einem Winkel zueinander verlaufen,
wobei der Referenzdetektor (45) auf der, in Richtung eines in den Empfänger (19') fallenden Strahls gesehen von der Empfänger-Linsenanordnung (21) weg weisenden Seite des zweiten Austrittsendes (61) angeordnet ist,
wobei der Analysefilter (43') zwischen dem ersten Eintrittsende (31) und der Empfänger-Linsenanordnung (21) angeordnet ist,
wobei der Analysefilter (43') derart ausgerichtet ist, dass entlang der Empfängerachse (23) auf den Analysefilter (43') fallendes und nicht-durchgelassenes Licht entlang der zweiten Stabachse (63) auf das zweite Eintrittsende (59) reflektiert wird und
wobei der Referenzfilter (49') zwischen der Empfänger-Linsenanordnung (21) und dem Analysefilter (43') angeordnet ist.

11. Gasdetektorsystem nach Anspruch 10, wobei die erste Stabachse (35) entlang der Empfängerachse (23) verläuft und
wobei die zweite Stabachse (63) unter einem Winkel, vorzugsweise senkrecht, zu der Empfängerachse (23) verläuft.

12. Gasdetektorsystem nach Anspruch 10 oder 11, wobei der Referenzfilter (49') derart ausgerichtet ist, dass entlang der Empfängerachse (23) auf den Referenzfilter (49') fallendes und nicht-durchgelassenes Licht auf eine in dem Empfänger (19') vorgesehene zweite positionssensitive Detektionseinrichtung (65) reflektiert wird.

13. Gasdetektorsystem nach einem der Ansprüche 10 bis 12, wobei die optischen Weglängen zwischen der Empfänger-Linsenanordnung (21) und dem ersten Lichtmischstab (29) und zwischen der Empfänger-Linsenanordnung (21) und dem zweiten Lichtmischstab (57) derart bemessen sind, dass der Brennpunkt (27, 27') innerhalb des ersten und des zweiten Lichtmischstabs (29, 57) liegt.

14. Gasdetektorsystem nach einem der Ansprüche 10 bis 13, wobei der Referenzfilter (49') als Bandpassfilter ausgebildet ist,
wobei der Analysefilter (43') als Kantenfilter ausgebildet ist und
wobei die untere Grenzwellenlänge des Bandpassfilters geringer als die Kantenwellenlänge ist.

15. Gasdetektorsystem nach einem der Ansprüche 1 bis 14, wobei der erste und/oder der zweite Lichtmischstab (29, 57) sich zum Austrittsende hin verjüngend konisch ausgebildet sind.

16. Gasdetektorsystem nach Anspruch 7 und/oder 12, wobei die erste und/oder zweite positionssensitive Detektionseinrichtung (17, 65) als CCD-, CMOS- oder Quadrantendetektor ausgebildet sind.

## Claims

1. A gas detector system comprising
a transmitter (1)
having a light source (3) from which an analytical light beam (5) is emitted, and
having a transmitter-lens arrangement (7) which is configured to collimate the analytical light beam (5) along an emission direction (9), and
a receiver (19, 19')
having a receiver-lens arrangement (21) which defines a receiver focal point (27, 27') and a receiver axis (23),
having an analytical detector (39) and a reference detector (45),
having an analytical filter (43, 43') which, viewed from the receiver-lens arrangement (21), is arranged in front of the analytical detector (39), and
having a reference filter (49, 49') which, viewed from the receiver-lens arrangement (21), is arranged in front of the reference detector (45),
**characterised in that** the receiver is equipped with a first light-mixing rod (29) which has a first inlet end (31) and a first outlet end (33) and defines a first rod axis (35) extending from the first inlet end (31) to the first outlet end (33), wherein the first inlet end (31) points to the receiver-lens arrangement (21), and
wherein the analytical detector (39) is arranged on the side of the first outlet end (33) pointing away from the receiver-lens arrangement (21).

2. A gas detector system according to claim 1, wherein the receiver-lens arrangement has a field lens (25) which is arranged in front of the inlet end (31).

3. A gas detector system according to claim 1 or 2,
wherein the focal point (27) lies within the first light-mixing rod (29).

4. A gas detector system according to one of claims 1 to 3, wherein the first rod axis (35) extends along the receiver axis (23),
wherein the first outlet end (33) has a prism arrangement (51) to split a beam leaving the first light-mixing rod (29) into a first beam (41) and a second beam (47),
wherein the first beam (41) propagates along a first direction, and the second beam (47) propagates along a second direction, and the first and second direction extend at an angle to the first rod axis (35),
wherein the analytical detector (39) is arranged along the first direction,
wherein the reference detector (45) is arranged along the second direction,
wherein the analytical filter (43) is arranged between the analytical detector (39) and the first outlet end (33), and
wherein the reference filter (49) is arranged between the reference detector (45) and the first outlet end (33).

5. A gas detector system according to claim 4, wherein the analytical filter (43) and the reference filter (49) are designed as bandpass filters whose transmission ranges differ from each other.

6. A gas detector system according to claim 4 or 5,
wherein a receiver light source (53) is arranged on the receiver axis (23) on the side of the first outlet end (33) pointing away from the receiver-lens arrangement (21).

7. A gas detector system according to claim 6, wherein the transmitter (1) has a filter (11) which is arranged between the transmitter-lens arrangement (7) and the light source (3) and reflects light, falling onto the transmitter-lens arrangement (7) in opposition to the emission direction, onto a first position-sensitive detection device (17) provided in the transmitter (1).

8. A gas detector system according to one of claims 1 to 7, wherein the first light-mixing rod (29) has a first circumferential wall (37) extending between the first inlet end (31) and the first outlet end (33), wherein light-sensitive sensors (55), preferably diodes, are arranged on the first circumferential wall (37) spaced apart in the circumferential direction, and wherein the light-sensitive region of the sensors (55) points towards the interior of the first light-mixing rod (29).

9. A gas detector system according to claim 8, wherein the first light-mixing rod (29) has a polygonal cross-section, viewed perpendicularly to the first rod axis (35).

10. A gas detector system according to claim 1 or 2, wherein the receiver (19') has a second light-mixing rod (57) which has a second inlet end (59) and a second outlet end (61) and defines a second rod axis (63) extending from the second inlet end (59) to the second outlet end (61), wherein the second inlet end (59), viewed in the direction of a beam falling into the receiver (19'), is arranged closer to the receiver-lens arrangement (21),
wherein the first and the second rod axis (35, 63) extend at an angle to each other,
wherein the reference detector (45) is arranged on the side of the second outlet end (61) pointing away from the receiver lens arrangement (21), viewed in the direction of a beam falling into the receiver (19'),
wherein the analytical filter (43') is arranged between the first inlet end (31) and the receiver-lens arrangement (21),
wherein the analytical filter (43') is aligned in such a way that light falling onto the analytical filter (43') along the receiver axis (23) and not transmitted is reflected along the second rod axis (63) onto the second inlet end (59), and
wherein the reference filter (49') is arranged between the receiver-lens arrangement (21) and the analytical filter (43').

11. A gas detector system according to claim 10, wherein the first rod axis (35) extends along the receiver axis (23), and
wherein the second rod axis (63) extends at an angle, preferably perpendicularly, to the receiver axis (23).

12. A gas detector system according to claim 10 or 11, wherein the reference filter (49') is aligned in such a way that light falling onto the reference filter (49') along the receiver axis (23) and not transmitted is reflected onto a second position-sensitive detection device (65) provided in the receiver (19').

13. A gas detector system according to one of claims 10 to 12, wherein the optical path lengths between the receiver-lens arrangement (21) and the first light-mixing rod (29) and between the receiver-lens arrangement (21) and the second light-mixing rod (57) are dimensioned so that the focal point (27, 27') lies within the first and the second light-mixing rod (29, 57)'.

14. A gas detector system according to one of claims 10 to 13, wherein the reference filter (49') is designed as a bandpass filter,
wherein the analytical filter (43') is designed as a cut-off filter, and
wherein the lower threshold wavelength of the bandpass filter is smaller than the cut-off wavelength.

15. A gas detector system according to one of claims 1 to 14, wherein the first and/or the second light-mixing rod (29, 57) are/is designed so as to be conical tapering towards the outlet end.

16. A gas detector system according to claim 7 and/or 12, wherein the first and/or second position-sensitive detection device (17, 65) are/is designed as a CCD detector, a CMOS detector or a quadrant detector.

## Revendications

1. Système de détection de gaz comprenant
un émetteur (1)
avec une source de lumière (3) par laquelle un faisceau lumineux d'analyse (5) est émis, et
avec un arrangement de lentilles d'émetteur (7) qui est conçu pour collimater le faisceau lumineux d'analyse (5) suivant une direction d'émission (9), et
un récepteur (19, 19')
avec un arrangement de lentilles de récepteur (21) qui définit un foyer de récepteur (27, 27') et un axe de récepteur (23),
avec un détecteur d'analyse (39) et un détecteur de référence (45),
avec un filtre d'analyse (43, 43') qui est disposé avant le détecteur d'analyse (39), vu de l'arrangement de lentilles de récepteur (21), et
avec un filtre de référence (49, 49') qui est disposé avant le détecteur de référence (45), vu de l'arrangement de lentilles de récepteur (21),
**caractérisé en ce que** le récepteur est équipé d'un premier barreau mélangeur de lumière (29) qui présente une première extrémité d'entrée (31) et une première extrémité de sortie (33) et définit un premier axe de barreau (35) s'étendant de la première extrémité d'entrée (31) à la première extrémité de sortie (33), la première extrémité d'entrée (31) étant dirigée vers l'arrangement de lentilles de récepteur (21) et le détecteur d'analyse (39) étant disposé du côté de la première extrémité de sortie (33) dirigé à l'opposé de l'arrangement de lentilles de récepteur (21).

2. Système de détection de gaz selon la revendication 1, dans lequel l'arrangement de lentilles de récepteur présente une lentille de champ (25) qui est disposée avant la première extrémité d'entrée (31).

3. Système de détection de gaz selon la revendication 1 ou la revendication 2, dans lequel le foyer (27) est situé à l'intérieur du premier barreau mélangeur de lumière (29).

4. Système de détection de gaz selon l'une des revendications 1 à 3, dans lequel le premier axe de barreau (35) s'étend suivant l'axe de récepteur (23),
la première extrémité de sortie (33) présentant un arrangement de prismes (51) pour diviser un faisceau qui quitte le premier barreau mélangeur de lumière (29) en un premier faisceau (41) et un deuxième faisceau (47),
le premier faisceau (41) se propageant suivant une première direction et le deuxième faisceau (47) suivant une deuxième direction, et la première et la deuxième direction s'étendant en faisant un angle avec le premier axe de barreau (35),
le détecteur d'analyse (39) étant disposé suivant la première direction,
le détecteur de référence (45) étant disposé suivant la deuxième direction,
le filtre d'analyse (43) étant disposé entre le détecteur d'analyse (39) et la première extrémité de sortie (33) et
le filtre de référence (49) étant disposé entre le détecteur de référence (45) et la première extrémité de sortie (33).

5. Système de détection de gaz selon la revendication 4, dans lequel le filtre d'analyse (43) et le filtre de référence (49) sont réalisés sous la forme de filtres passe-bande dont les bandes passantes sont différentes.

6. Système de détection de gaz selon la revendication 4 ou la revendication 5, dans lequel une source de lumière de récepteur (53) est disposée sur l'axe de récepteur (23) du côté de la première extrémité de sortie (33) dirigée à l'opposé de l'arrangement de lentilles de récepteur (21).

7. Système de détection de gaz selon la revendication 6, dans lequel l'émetteur (1) présente un filtre (11) disposé entre l'arrangement de lentilles d'émetteur (7) et la source de lumière (3), qui réfléchit la lumière incidente sur l'arrangement de lentilles d'émetteur (7) dans la direction opposée à la direction d'émission sur un premier dispositif de détection (17) sensible à la position prévu dans l'émetteur (1).

8. Système de détection de gaz selon l'une des revendications 1 à 7, dans lequel le premier barreau mélangeur de lumière (29) présente une première paroi périphérique (37) s'étendant entre la première extrémité d'entrée (31) et la première extrémité de sortie (33),
des capteurs photosensibles (55), de préférence des diodes, étant disposés de manière espacée en direction périphérique sur la première paroi périphérique (37) et
la zone photosensible des capteurs (55) étant dirigée vers l'intérieur du premier barreau mélangeur de lumière (29).

9. Système de détection de gaz selon la revendication 8, dans lequel le premier barreau mélangeur de lumière (29) présente une section transversale polygonale, vu perpendiculairement au premier axe de barreau (35).

10. Système de détection de gaz selon la revendication 1 ou la revendication 2, dans lequel le récepteur (19') présente un deuxième barreau mélangeur de lumière (57), qui présente une deuxième extrémité d'entrée (59) et une deuxième extrémité de sortie (61) et définit un deuxième axe de barreau (63) s'étendant de la deuxième extrémité d'entrée (59) à la deuxième extrémité de sortie (61), la deuxième extrémité d'entrée (59) étant disposée plus près de l'arrangement de lentilles de récepteur (21), vu dans la direction d'un faisceau incident dans le récepteur (19'), le premier et le deuxième axe de barreau (35, 63) s'étendant en faisant un angle entre eux,
le détecteur de référence (45) étant disposé du côté de la deuxième extrémité de sorties (61) dirigée à l'opposé de l'arrangement de lentilles de récepteur (21), vu dans la direction d'un faisceau incident dans le récepteur (19'),
le filtre d'analyse (43') étant disposé entre la première extrémité d'entrée (31) et l'arrangement de lentilles de récepteur (21),
le filtre d'analyse (43') étant orienté de façon que la lumière incidente sur le filtre d'analyse (43') suivant l'axe de récepteur (23) et non admise à passer soit réfléchie sur la deuxième extrémité d'entrée (59) le long du deuxième axe de barreau (63) et
le filtre de référence (49') étant disposé entre l'arrangement de lentilles de récepteur (21) et le filtre d'analyse (43').

11. Système de détection de gaz selon la revendication 10, dans lequel le premier axe de barreau (35) s'étend suivant l'axe de récepteur (23) et
dans lequel le deuxième axe de barreau (63) s'étend en faisant un angle, de préférence perpendiculairement, par rapport à l'axe de récepteur (23).

12. Système de détection de gaz selon la revendication 10 ou la revendication 11, dans lequel le filtre de référence (49') est orienté de façon que la lumière incidente sur le filtre de référence (49') suivant l'axe de récepteur (23) et non admise à passer soit réfléchie sur un deuxième dispositif de détection (65) sensible à la position prévu dans le récepteur (19').

13. Système de détection de gaz selon l'une des revendications 10 à 12, dans lequel les longueurs de trajet optique entre l'arrangement de lentilles de récepteur (21) et le premier barreau mélangeur de lumière (29) et entre l'arrangement de lentilles de récepteur (21) et le deuxième barreau mélangeur de lumière (57) sont dimensionnées de façon que le foyer (27, 27') se situe à l'intérieur du premier et du deuxième barreau mélangeur de lumière (29, 57).

14. Système de détection de gaz selon l'une des revendications 10 à 13, dans lequel le filtre de référence (49') est réalisé sous la forme d'un filtre passe-bande,
le filtre d'analyse (43') est réalisé sous la forme d'un filtre à arête et
la longueur d'onde limite inférieure du filtre passe-bande est inférieure à la longueur d'onde d'arête.

15. Système de détection de gaz selon l'une des revendications 1 à 14, dans lequel le premier et/ou le deuxième barreau mélangeur de lumière (29, 57) sont de forme conique se rétrécissant vers l'extrémité de sortie.

16. Système de détection de gaz selon la revendication 7 et/ou la revendication 12, dans lequel le premier et/ou le deuxième dispositif de détection sensible à la position (17, 65) sont réalisés sous la forme d'un détecteur CCD, CMOS ou à quadrant.
